**Europäisches Patentamt**

**European Patent Office**

**Office européen des brevets**

(19)

(11) Veröffentlichungsnummer : **0 142 090 B1**

## EUROPÄISCHE PATENTSCHRIFT

(12)

(45) Veröffentlichungstag der Patentschrift :
15.06.88

(21) Anmeldenummer : 84112986.9

(22) Anmeldetag : 27.10.84

(51) Int. Cl.⁴ : **C 07 C 29/38, C 07 C 31/22**

(54) Verfahren zur Herstellung von Trimethylolalkanen aus Alkanalen und Formaldehyd.

(30) Priorität : 11.11.83 DE 3340791

(43) Veröffentlichungstag der Anmeldung :
22.05.85 Patentblatt 85/21

(45) Bekanntmachung des Hinweises auf die Patenterteilung : 15.06.88 Patentblatt 88/24

(84) Benannte Vertragsstaaten :
BE DE FR GB IT NL

(56) Entgegenhaltungen :
Keine Entgegenhaltungen

(73) Patentinhaber : BASF Aktiengesellschaft
Carl-Bosch-Strasse 38
D-6700 Ludwigshafen (DE)

(72) Erfinder : Merger, Franz, Dr.
Max-Slevogt-Strasse 25
D-6710 Frankenthal (DE)
Erfinder : Hettinger, Peter, Dr.
Schloss-Strasse 3
D-6803 Edingen-Neckarhausen (DE)
Erfinder : Hupfer, Leopold, Dr.
Waltershoehe 3
D-6701 Friedelsheim (DE)
Erfinder : Paetsch, Juergen, Dr.
Am Altenbach 30
D-6706 Wachenheim (DE)

## Beschreibung

Diese Erfindung betrifft ein Verfahren zur Herstellung von Trimethylolalkanen durch Umsetzung von n-Alkanalen mit Formaldehyd und Trialkylaminen.

Trimethylolpropan kann man bekanntlich durch Umsetzung von n-Butyraldehyd mit Formaldehyd in wäßrigem Medium in Gegenwart eines alkalisch wirkenden Mittels, wie Natriumhydroxid, herstellen. Das bei diesem, z. B. in der DE-AS 11 54 080 beschriebenen Verfahren als Zwischenprodukt entstehende Dimethylolbutanal reagiert mit Formaldehyd und dem Alkali nach Cannizzaro zu Trimethylpropan und Formiat. Nachteilig ist bei diesem Verfahren, daß mindestens stöchiometrische Mengen an Formiaten als Nebenprodukte anfallen. Die Salze müssen wegen ihrer zersetzenden Wirkung vor der Reindestillation der Trimethylolalkane vollständig abgetrennt werden, wodurch sich erhebliche Umweltprobleme ergeben.

Die Herstellung von reinem Trimethylolpropan ist auf diesem Wege nicht in befriedigender Weise möglich, da erhebliche Mengen an Formaldehyd in Gegenwart des basischen wirkenden Mittels mit dem Alkanal eine Cannizzaro-Reaktion eingehen und Gemische schwer trennbarer Komponenten gebildet werden.

In der DE-OS 19 52 738 wird ein Verfahren zur Herstellung von Trimethylolpropan beschrieben, bei dem man n-Butyraldehyd mit wäßrigem Formaldehyd in Gegenwart tertiärer organischer Amine umsetzt. Bei diesem Verfahren wendet man, bezogen auf n-Butyraldehyd, die mehr als fünffache molare Menge Formaldehyd an. Man gibt das tertiäre Amin mit dem Ziel der einfacheren Salzabtrennung in einer überstöchiometrischen Menge zu. Der Formaldehyd-Überschuß wird durch eine technisch aufwendige Druckdestillation entfernt. Auch im Hinblick auf Ausbeute und Reinheit des Trimethylolpropans ist das Verfahren unbefriedigend.

Nach den Angaben der DE-AS 25 07 461 läßt sich Trimethylolpropan dadurch herstellen, daß man n-Butyraldehyd mit Formaldehyd in Gegenwart katalytischer Mengen spezieller tertiärer verzweigter Alkylamine, wie Dimethylaminoneopentanol, umsetzt, das so erhaltene Dimethylolbutanal durch Destillation reinigt und katalytisch hydriert. Nach diesem Verfahren werden unbefriedigende Ausbeuten an Trimethylolpropan erzielt. Ersetzt man bei diesem Verfahren den basischen Katalysator z. B. durch Triethylamin, so fallen die Ausbeuten stark ab (s. DE-AS 25 07 461, Vergleichsbeispiele 2 und 3).

Bessere Resultate mit Trialkylaminen als Katalysator konnten entsprechend der Lehre der DE-OS 28 13 201 nur mit einem hohen Formaldehyd-Überschuß erhalten werden. Bei diesem Verfahren ist es erforderlich, bezogen auf den n-Butyraldehyd, die 5- bis 30-molare Menge Formaldehyd und die 0,01- bis 0,5-molare Menge Trialkylamin anzuwenden. Für eine wirtschaftliche Herstellung von Trimethylolpropan ist dieses Verfahren wenig geeignet, da der große Formaldehyd-Überschuß vor der katalytischen Hydrierung abgetrennt werden muß. Außerdem befriedigen die Ausbeuten nicht.

Da die Produktion von Trimethylolalkanen unter Verwendung eines so hohen Formaldehyd-Überschusses aus wirtschaftlichen Gründen die Rückgewinnung von Formaldehyd und einen damit verbundenen erheblichen technischen Aufwand erforderlich macht, war nach einem Verfahren zu suchen, das es gestattet, Trimethylolalkane in guter Ausbeute und hoher Reinheit auf wirtschaftlich einfachere Weise herzustellen.

Nach dem Verfahren der Erfindung, das diese vorteilhafte Herstellung ermöglich, wird bei der Gewinnung von Trimethylolalkanen durch Umsetzung von n-Alkanalen mit Formaldehyd und Trialkylaminen in wäßriger Lösung bei Temperaturen bis 120 °C und nachfolgende Hydrierung so verfahren, daß man, bezogen auf 1 Mol des Alkanals, 2,2 bis 4,5 Mol Formaldehyd und 0,6 bis 3 Mol Trialkylamin anwendet, und das Reaktionsgemisch nach oder vor der Hydrierung destillativ aufarbeitet.

Als n-Alkanale kommen z. B. in Betracht 3-Ethyl-, 3-n-Propyl-, 3-Isopropyl-, 3-n-Butyl-, 3-Isobutyl-, 3-sek.-Butyl-, 3-tert.-Butyl-butanal sowie entsprechende 8 Kohlenstoffatome enthaltende n-Pentanale, n-Hexanale ; 4-Ethyl-, 4-n-Propyl-, 4-Isopropyl-pentanale, 4-Ethyl-n-hexanale ; 5-Ethyl-n-hexanale ; 3-Methylhexanal, 3-Methyl-heptanal ; 4-Methyl-pentanal, 4-Methyl-heptanal, 5-Methyl-hexanal, 5-Methyl-heptanal ; 3,3,5-Trimethyln-pentyl-, 3,3-Dimethyl-n-butyl-, 3,3-Dimethyl-n-pentyl-, 4,4-Dimethylhexyl-, 4,5-Dimethylhexyl-, 3,4-Dimethylhexyl-, 3,5-Dimethylhexyl-, 3,3-Dimethylhexyl-, 3-Methyl-4-ethylpentyl-, 3,3,4-Trimethylpentyl-, 3,4,4-Trimethylpentyl-aldehyd ; bevorzugt sind Propanal, n-Butanal, n-Pentanal, 3-Methylbutanal, n-Hexanal, 3-Methylpentanal, n-Heptanal, 4-Methylhexanal, n-Octanal.

Als Trialkylamine kommen z. B. Trimethylamin, Triethylamin, Tri-n-propylamin oder Tri-n-butylamin in Betracht. Bezogen auf 1 Mol des Alkanals werden 2,2 bis 4,5 Mol, vorzugsweise 2,5 bis 4 und insbesondere 2,75 bis 3,5 Mol Formaldehyd und 0,6 bis 3 Mol, vorzugsweise 0,7 bis 2 und insbesondere 0,8 bis 1,5 Mol Trialkylamin eingesetzt.

Die Umsetzung wird im allgemeinen bei Temperaturen bis 120 °C, vorzugsweise von 20 bis 100 °C, insbesondere bei 40 bis 80 °C, durchgeführt. Man arbeitet mit Unterdruck, Überdruck oder drucklos, vorzugsweise drucklos, diskontinuierlich oder kontinuierlich. Gegebenenfalls verwendet man zusätzlich unter den Reaktionsbedingungen inerte, organische Lösungsmittel, vorzugsweise mit Wasser gut mischbare Lösungsmittel, vorteilhaft cyclische Ether, z. B. Tetrahydrofuran, Dioxan ; Ester wie Methylacetat, Ethylacetat, Methylpropionat ; Alkanole wie Ethanol, Metha-

nol, Glykol, Ethylenglykolmonoethylether, Methylglykol.

Die Reaktionszeiten betragen etwa 0,5 bis 24 Stunden, vorzugsweise 1 bis 10, insbesondere 1 bis 7 Stunden.

Der Formaldehyd wird zweckmäßigerweise als wäßrige Lösung, z. B. als 10- bis 50 Gew.%ige wäßrige Lösung eingesetzt. Der Wassergehalt des wäßrigen Reaktionsgemisches liegt z. B. bei 50 bis 85 Gew.%, vorzugsweise bei 60 bis 80 Gew.% und insbesondere bei 65 bis 75 Gew.%.

Das Umsetzungsgemisch wird vor oder nach der Hydrierung destillativ aufgearbeitet. Man arbeitet destillativ auf, indem man im ersten Falle aus dem Reaktionsgemisch, zweckmäßig unter vermindertem Druck, zunächst Wasser und dann freies Trialkylamin sowie das bei der Umsetzung entstandene Trialkylammoniumformiat abdestilliert. Den Rückstand nimmt man in Wasser auf und unterwirft das wäßrige Gemisch der Hydrierung. Man kann das wäßrige Reaktionsgemisch aber auch vor der destillativen Aufarbeitung oder nach einer nur teilweise destillativen Entfernung der genannten leichter flüchtigen Bestandteile hydrieren.

Die Hydrierung nimmt man auf an sich übliche Weise, zweckmäßigerweise in wäßriger Lösung bei Temperaturen von 80 bis 150 °C und im Druckbereich von 20 bis 200 bar vor. Dabei verwendet man an sich übliche Hydrierkatalysatoren, wie solche, die Nickel, Kupfer oder Edelmetalle, wie Platin oder Palladium enthalten. Die Katalysatoren können Trägerkatalysatoren sein und in der Suspensions- oder Festbettfahrweise angewendet werden. Besonders geeignete Katalysatoren der genannten Art werden z. B. in der DE-OS 30 27 890 beschrieben.

Nach der Hydrierung arbeitet man die hydrierte wäßrige Lösung z. B. unmittelbar oder nach Extraktion mit Lösungsmitteln, das sind z. B. in Wasser schwerlösliche Alkohole, Ketone oder Ester, durch Destillation oder Kristallisation auf. Man erhält die Trimethylolalkane in sehr guter Ausbeute und Reinheit.

### Beispiel 1

216 g (3 Mol) n-Butyraldehyd, 1 200 g Formalinlösung (30 %ig) (12 Mol formaldehyd), 450 g (4,5 Mol) Triethylamin und 1 000 g Wasser werden zusammengegeben. Das Gemisch hält man 4 Std. auf 70 °C. Anschließend werden unter vermindertem Druck (20 mbar) zunächst das Wasser sowie freies Triethylamin und dann das bei der Reaktion entstandene Triethylammoniumformiat ($Sdp_{20}$: 110 °C) abdestilliert. Der Rückstand wird mit der gleichen Gewichtsmenge Wasser versetzt und bei 120 °C und 60 bar einer kontinuierlichen Hydrierung unterzogen (Hydrierkontakt bestehend aus $CuO$ und $Al_2O_3$ mit einem Atomverhältnis von Cu zu Al wie 0,75 zu 1). Nach destillativer Aufarbeitung isoliert man 342 g Trimethylolpropan (TMP-$Sdp_3$: 153 °C) in 85 %iger Ausbeute, bezogen auf n-Butyraldehyd, neben 28 g Di-Trimethylolpropan (Di-TMP-$Sdp_3$ = 185 °C) in 7,6 %iger Ausbeute, bezogen auf n-Butyraldehyd.

### Beispiel 2

Zu einer Mischung von 450 g (4,5 Mol) Triethylamin, 1 200 g Formalinlösung (30 %ig) (12 Mol Formaldehyd) und 1 000 g Wasser tropft man innerhalb von 45 min 216 g (3 Mol) n-Butyraldehyd. Danach wird die Lösung 4 Std. auf 70 °C erhitzt. Nach destillativer Abtrennung von Wasser, Triethylamin und Triethylammoniumformiat unter vermindertem Druck (entsprechend Beispiel 1) wird der Rückstand mit der vierfachen Gewichtsmenge Wasser verdünnt und bei 100 °C, 100 bar im Autoklaven in Gegenwart von 24 g Pd-Kohle (10 %ig) hydriert. Nach 3 Std. ist die Wasserstoffaufnahme beendet. Nach Abfiltrieren des katalysators und Abtrennung der Niedersieder durch Destillation werden bei 3 mbar 334 g Trimethylolpropan ($Sdp_3$: 153 °C, Ausbeute 83 %, bezogen auf n-Butyraldehyd) sowie 26 g Di-Trimethylolpropan ($Sdp_3$: 185 °C, Ausbeute 7 %, bezogen auf n-Butyraldehyd) destillativ isoliert.

### Beispiel 3

Zu einer Mischung aus 700 g Formalinlösung (15 %ig) (3,5 Mol Formaldehyd) und 150 g (1,5 Mol) Triethylamin werden innerhalb von 20 min 72 g (1 Mol) n-Butyraldehyd getropft. Die Mischung wird dann 5 Std. bei 73 °C gerührt. Die homogene Lösung wird bei 120 °C, 50 bar einer kontinuierlichen Hydrierung zugeführt (Hydrierkontakt bestehend aus 81,47 % NiO ; 18,53 % $Al_2O_3$) und der Reaktoraustrag bei vermindertem Druck destilliert. Nach Abtrennung von Niedersiedern und Triethylammoniumformiat bei 2,0 mbar werden 110 g Trimethylolpropan (Ausbeute 82 %, bezogen auf n-Butyraldehyd) und 18 g Di-Trimethylolpropan (Ausbeute 14 %, bezogen auf n-Butyraldehyd) durch Vakuumdestillation gemäß Beispiel 2 isoliert.

### Beispiel 4

Man leitet in eine kontinuierliche Reaktionskaskade, die aus drei hintereinandergeschalteten Kolben mit je 900 ml Reaktionsvolumen besteht, stündlich 98,7 ml (0,71 Mol) Triethylamin, 42,9 ml (0,49 Mol) n-Butyraldehyd und 308,7 ml 15,2 %ige Formaldehydlösung (1,7 Mol Formaldehyd) ein, wobei die Reaktionstemperatur in allen drei Kolben 80 °C beträgt. Der Kaskadenaustrag wird einer kontinuierlichen Hydrierapparatur zugeführt und bei 115 °C, 60 bar hydriert (Kontakt : 81,47 % NiO ; 18,53 % $Al_2O_3$). Anschließend werden Niedersieder und im Verlauf der Reaktion gebildetes Triethylammonium-formiat durch Vakuumdestillation abgetrennt (Druck : 20 mbar). Der Rückstand liefert durch fraktionierende Destillation bei 3 mbar 125 g Trimethylolpropan/l Hydrieraustrag (Ausbeute 84 %, bezogen auf n-Butyraldehyd) sowie 18 g Di-Trimethylolpropan/l Hydrieraustrag (Ausbeute 13 %, bezogen auf n-Butyraldehyd).

### Beispiel 5

Man tropft unter Rührer in eine Mischung von 550 g Formalinlösung (30 %ig) (5,5 Mol Formaldehyd), 302 g (3 Mol) Triethylamin und 550 g Wasser innerhalb von 30 min 144 g (2 Mol) n-Butyraldehyd. Anschließend erhitzt man 4 Std. auf 72 °C. Die Reaktionsmischung wird dann bei 130 °C, 50 bar kontinuierlich hydriert (Kontakt : 81,47 %NiO, 18,53 % $Al_2O_3$). Anschließend werden die Niedersieder sowie gebildetes Triethylammoniumformiat durch Vakuumdestillation bei 20 mbar abgetrennt. Durch fraktionierende Destillation des Rückstands erhält man 203 g Trimethylolpropan vom $Sdp_3$ = 153 °C (Ausbeute 76 %, bezogen auf b-Butyraldehyd) sowie 40 g Di-trimethylolpropan vom $Sdp_3$ = 185 °C (16 %, bezogen auf n-Butyraldehyd).

**Patentansprüche**

1. Verfahren zur Herstellung von Trimethylolalkanen durch Umsetzung von n-Alkanalen mit Formaldehyd und Trialkylaminen in wäßriger Lösung bei Temperaturen bis 120 °C und nachfolgende Hydrierung, dadurch gekennzeichnet, daß man, bezogen auf 1 Mol des Alkanals, 2,2 bis 4,5 Mol Formaldehyd und 0,6 bis 3 Mol Trialkylamin anwendet, und das Reaktionsgemisch nach oder vor der Hydrierung destillativ aufarbeitet.

2. Verfahren nach Anspruch 1, dadurch gekennzeichnet, daß man, bezogen auf 1 Mol des Alkanals 2,5 bis 4 Mol Formaldehyd und 0,7 bis 2 Mol Trialkylamin anwendet.

3. Verfahren nach Anspruch 1, dadurch gekennzeichnet, daß man, bezogen auf 1 Mol des Alkanals 2,75 bis 3,5 Mol Formaldehyd und 0,8 bis 1,5 Mol Trialkylamin anwendet.

4. Verfahren nach Anspruch 1, dadurch gekennzeichnet, daß man das Reaktionsgemisch unmittelbar nach der Umsetzung hydriert.

**Claims**

1. A process for the preparation of a trimethylolalkane by reacting an n-alkanal with formaldehyde and a trialkylamine in aqueous solution at as high as 120 °C and then hydrogenating the product, wherein from 2.2 to 4.5 moles of formaldehyde and from 0.6 to 3 moles of trialkylamine are used per mole of the alkanal, and the reaction mixture is worked up by distillation either before or after the hydrogenation.

2. A process as claimed in claim 1, wherein from 2.5 to 4 moles of formaldehyde and from 0.7 to 2 moles of trialkylamine are used per mole of the alkanal.

3. A process as claimed in claim 1, wherein from 2.75 to 3.5 moles of formaldehyde and from 0.8 to 1.5 moles of trialkylamine are used per mole of the alkanal.

4. A process as claimed in claim 1, wherein the reaction mixture is hydrogenated directly after the reaction.

**Revendications**

1. Procédé de préparation de triméthylolalcanes par réaction de n-alcanals avec du formaldéhyde et des trialkylamines, en solution aqueuse à des températures allant jusqu'à 120 °C, et par hydrogénation subséquente, caractérisé en ce qu'on utilise, pour 1 mole de l'alcanal, 2,2 à 4,5 moles de formaldéhyde et 0,6 à 3 moles de trialkylamine, et en ce qu'on traite le mélange réactionnel par distillation après ou avant l'hydrogénation.

2. Procédé suivant la revendication 1, caractérisé en ce qu'on utilise, pour 1 mole de l'alcanal, 2,5 à 4 moles de formaldéhyde et 0,7 à 2 moles de trialkylamine.

3. Procédé suivant la revendication 1, caractérisé en ce qu'on utilise, pour 1 mole de l'alcanal, 2,75 à 3,5 moles de formaldéhyde et 0,8 à 1,5 mole de trialkylamine.

4. Procédé suivant la revendication 1, caractérisé en ce qu'on hydrogène le mélange réactionnel directement après ladite réaction.